(19) Europäisches Patentamt
European Patent Office
Office européen des brevets

(11) Veröffentlichungsnummer: **0 094 555**
**A1**

(12) # EUROPÄISCHE PATENTANMELDUNG

(21) Anmeldenummer: 83104373.2

(22) Anmeldetag: 04.05.83

(51) Int. Cl.³: C 07 D 231/06
C 07 D 409/04, C 07 D 401/04
C 07 D 417/04, C 07 D 231/12
//C07D307/32, C07D409/06,
C07D405/06, A61K31/415

(30) Priorität: 07.05.82 CH 2825/82

(43) Veröffentlichungstag der Anmeldung:
23.11.83 Patentblatt 83/47

(84) Benannte Vertragsstaaten:
AT BE CH DE FR GB IT LI LU NL SE

(71) Anmelder: Byk Gulden Lomberg Chemische Fabrik GmbH
Byk-Gulden-Strasse 2
D-7750 Konstanz(DE)

(72) Erfinder: Kohl, Bernhard, Dr.
Heinrich-von-Tettingenstrasse 35a
D-7750 Konstanz 21(DE)

(54) **Substituierte 2-Pyrazolin-4-essigsäuren, ein Verfahren zu ihrer Herstellung, ihre Verwendung und sie enthaltende Mittel.**

(57) 1,3-Disubstituierte 2-Pyrazolin-4-essigsäuren der allgemeinen Formel I

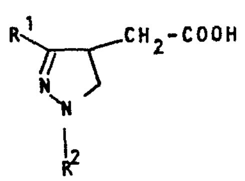

(I),

worin $R^1$ und $R^2$ gleich oder verschieden sind und eine gegebenenfalls substituierte Arylgruppe oder eine gegebenenfalls substituierte Heteroarylgruppe bedeuten, und ihre Salze mit anorganischen oder organischen Basen sind neue Verbindungen. Sie können als optische Aufheller oder Fluoreszenzfarbstoffe eingesetzt werden. Bestimmte 2-Pyrazolin-4-essigsäuren können auch als Zwischenprodukte für die Herstellung von entsprechenden Pyrazol-4-essigsäuren dienen. Die 2-Pyrazolin-4-essigsäuren der allgemeinen Formel I und ihre Salze werden durch Umsetzung eines Lactons der allgemeinen Formel II

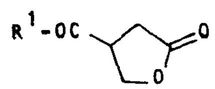

(II),

worin $R^1$ die oben angegebene Bedeutung hat, mit einem Hydrazin der allgemeinen Formel III $R^2 - NH - NH_2$ (III), worin $R^2$ die oben angegebene Bedeutung hat, erhalten.

EP 0 094 555 A1

Croydon Printing Company Ltd

Die Erfindung betrifft substituierte 2-Pyrazolin-4-essigsäuren, ein Verfahren zu ihrer Herstellung, ihre Verwendung und sie enthaltende Mittel.

Substituierte 2-Pyrazoline, ihre Herstellung und Verwendung werden von Wagner et al. in Angew. Chem. 78(1966)769-774 beschrieben. Es wurden nun 2-Pyrazolin-4-essigsäuren und ein neues Verfahren zur Herstellung dieser Verbindungen entwickelt.

Gegenstand der Erfindung sind 1,3-disubstituierte 2-Pyrazolin-4-essigsäuren der allgemeinen Formel I

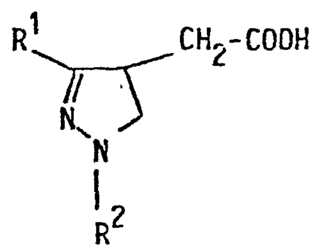

(I),

worin

$R^1$ eine gegebenenfalls substituierte Arylgruppe oder eine gegebenenfalls substituierte Heteroarylgruppe und

$R^2$ eine gegebenenfalls substituierte Arylgruppe oder eine gegebenenfalls substituierte Heteroarylgruppe bedeuten,

und ihre Salze mit anorganischen oder organischen Basen.

Arylgruppen sind solche, die 6 bis 12 Kohlenstoffatome enthalten. Bei dieser Angabe der Zahl der Kohlenstoffatome werden die Substituenten am Arylrest nicht gezählt. Als Beispiele seien die Phenyl-, Biphenylyl- und Naphthylgruppe genannt.

Heteroarylgruppen sind solche, die 3 bis 9 Kohlenstoffatome und 1 oder 2 Stickstoffatome oder 1 Sauerstoffatom oder 1 Schwefelatom oder 1 Stickstoffatom und 1 Sauerstoffatom oder 1 Stickstoffatom und 1 Schwefelatom enthalten. Bei dieser Angabe der Zahl der Kohlenstoffatome werden die Substituenten am Heteroarylrest nicht gezählt. Beispiele für Heteroarylreste sind der Thienyl-, Furyl-, Pyrazolyl-, Benzthiazolyl- und Pyridylrest.

Substituenten an der Aryl- bzw. Heteroarylgruppe sind 1 oder 2 Niederalkyl-, Niederalkoxy-, Niederalkylthio- oder Trifluormethylreste und/ oder 1 oder 2 Halogenatome. Substituenten an der Arylgruppe sind ferner eine Carboxy-, eine gegebenenfalls durch Niederalkyl mono- oder disubstituierte Carboxamid-, eine Sulfonsäure-, eine gegebenenfalls substituierte Sulfonamid- oder eine gegebenenfalls substituierte Niederalkylsulfonylgruppe. Niederalkyl-, Niederalkoxy-, Niederalkylthio-Substituenten sind solche mit 1 bis 4 Kohlenstoffatomen. Halogenatome sind Fluor-, Chlor- und Bromatome.
Als Salze kommen beliebige Salze in Betracht. Als Kationen für die Salzbildung werden vor allem Alkalimetall-, Erdalkalimetall- und Erdmetallionen verwendet, oder das Ammoniumionen, aber auch die korrespondierenden Kationensäuren ein- oder mehrsäuriger organischer Stickstoffbasen, insbesondere organischer Amine.

Beispielsweise werden die Kationen der Metalle Lithium, Natrium, Kalium, Magnesium, Calcium oder Aluminium oder die Kationensäuren von Ethanolamin, Diethanolamin, Triethanolamin, Ethylendiamin, Dimethylamin, Diethylamin, Morpholin, Piperazin, Methylcyclohexylamin, Glucosamin, N-Methylglucamin, N-Methylglucosamin, ferner von tert.-Butylamin, Dibutylamin, Diisopropylamin, Triethylamin, Isopropylamin, Chinolin und Ammoniak verwendet.

Eine Ausgestaltung der Erfindung sind 1,3-disubstituierte 2-Pyrazolin-4-essigsäuren der allgemeinen Formel I*

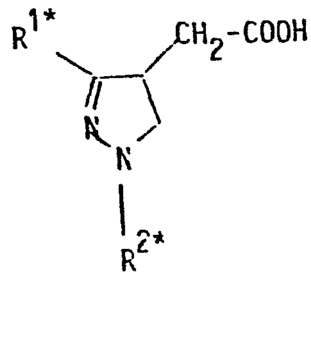

(I*),

worin

$R^{1*}$ einen Phenylrest, einen durch $R^{3*}$ substituierten Phenylrest, einen 4-Biphenylyl-Rest oder einen 2-Naphthylrest,

$R^{2*}$ einen durch $R^{4*}$ substituierten Phenylrest oder einen Benzthiazolylrest,

$R^{3*}$ ein oder zwei in 3- und/oder 4-Stellung ständige Halogenatome und

$R^{4*}$ eine in 4-Stellung ständige Carboxy-, Sulfo-, Aminosulfonyl-, Dimethylaminoniederalkylaminosulfonyl-, Niederalkylsulfonyl- oder eine ω-Sulfoniederalkylsulfonylgruppe bedeuten,

und ihre Salze.

Bevorzugte Verbindungen der Ausgestaltung I* sind solche, in denen $R^{1*}$ einen Phenylrest oder einen durch $R^{3*}$ substituierten Phenylrest, $R^{2*}$ einen durch $R^{4*}$ substituierten Phenylrest, $R^{3*}$ ein in 4-Stellung ständiges Chloratom oder zwei in 3- und 4-Stellung ständige Chloratome und $R^{4*}$ eine Carboxy-, eine Sulfo-, eine Aminosulfonyl-, eine Methylsulfonyl- oder eine 2-Sulfoethylsulfonylgruppe bedeuten, und ihre Salze.

Eine weitere Ausgestaltung der Erfindung sind 1,3-disubstituierte 2-Pyrazolin-4-essigsäuren der allgemeinen Formel I**

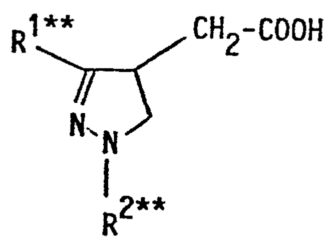

(I**),

worin

$R^{1**}$ einen Phenylrest, einen durch $R^{5**}$ substituierten Phenylrest, einen 4-Biphenylylrest, einen 2-Naphthylrest, einen 2-Thienylrest, einen 2-Furyl- oder einen 2- oder 3-Pyridylrest,

$R^{2**}$ einen Phenylrest, einen 2-Thienylrest, einen 2-Furyl- oder einen 2- oder 3-Pyridylrest,

$R^{5**}$ ein in 3- oder 4-Stellung ständiges Chlor- oder Fluoratom, eine in 3- oder 4-Stellung ständige Methyl- oder Methoxygruppe oder eine in 3-Stellung ständige Trifluormethylgruppe bedeuten,

und ihre Salze.

Bevorzugte Verbindungen der Ausgestaltung I** sind solche, in denen $R^{1**}$ einen Phenylrest, einen durch $R^{5**}$ substituierten Phenylrest oder einen 2-Thienylrest, $R^{2**}$ einen Phenylrest oder einen 2-Thienylrest und $R^{5**}$ ein in 4-Stellung ständiges Chloratom bedeuten, und ihre Salze. Besonders bevorzugt ist die Verbindung der Ausgestaltung I**, in der $R^{1**}$ einen 4-Chlorphenylrest und $R^{2**}$ einen Phenylrest bedeuten, und ihre Salze.

Ein weiterer Gegenstand der Erfindung ist ein Verfahren zur Herstellung von 2-Pyrazolin-4-essigsäuren der allgemeinen Formel I und ihren Salzen, das dadurch gekennzeichnet ist, daß man ein Lacton der allgemeinen Formel II

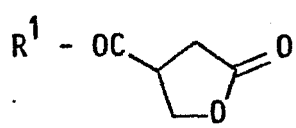 (II),

worin $R^1$ die oben angegebene Bedeutung hat, mit einem Hydrazin der allgemeinen Formel III

$$R^2 - NH - NH_2 \qquad (III),$$

worin $R^2$ die oben angegebene Bedeutung hat, umsetzt und gegebenenfalls anschließend die erhaltene Säure der allgemeinen Formel I in ein Salz oder ein erhaltenes Salz in die freie Säure überführt.

Die Umsetzung des Lactons II mit dem Hydrazin III wird in einem protischen Lösungsmittel durchgeführt, wobei der pH-Wert in einem breiten Bereich variiert werden kann. Im allgemeinen wird die Reaktion bei pH 3 bis 10, vorzugsweise pH 4 bis 6 ausgeführt. Das Gewichtsverhältnis Lösungsmittel zu Lacton II beträgt (3 bis 20):1, vorzugsweise (8 bis 12):1. Das molare Verhältnis Lacton II zu Hydrazin III beträgt zweckmäßig 3:1 bis 1:3, bevorzugt wird das Hydrazin III im geringen Überschuß eingesetzt. Als Lösungsmittel (Lösungsmittelgemische) kommen beispielsweise in Betracht Essigsäure; Essigsäure/Pyridin; Essigsäure/Natriumacetat/Wasser; Dioxan/NaOH; Wasser/NaOH; Alkanole/NaOH(wie Ethanol/NaOH); Dichlorethan/Essigsäure/Pyridin; Puffer (wie

Zitronensäurepuffer von pH 4,5), gegebenenfalls unter Zusatz von Dioxan als Lösungsvermittler.

Die Reaktionstemperaturen liegen zwischen Raumtemperatur und der Rückflußtemperatur des Lösungsmittels, bevorzugt zwischen 80°C und der Rückflußtemperatur des Lösungsmittels. Die Reaktionszeiten betragen bei technischen Ansätzen 5 bis 70, vorzugsweise 40 bis 60 Stunden. Die Reaktionszeit kann gewünschtenfalls durch Arbeiten unter Druck bei 1 bis 50 at (1 bis 50 bar), vorzugsweise 1 bis 10 at (1 bis 10 bar) bei Temperaturen über dem Siedepunkt des Lösungsmittels, beispielsweise 130 bis 200° verringert werden.

Zur Herstellung der 2-Pyrazolin-4-essigsäuren der Ausgestaltungen I* bzw. I** werden entsprechende Ausgangsverbindungen II* bzw. II** und III* bzw. III**

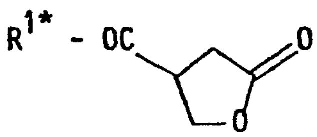 (II*), 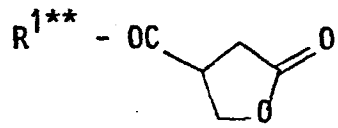 (II**),

$R^{2*}$ - NH - $NH_2$ (III*), $R^{2**}$ - NH - $NH_2$ (III**),

worin $R^{1*}$, $R^{1**}$, $R^{2*}$ und $R^{2**}$ die oben angegebene Bedeutung haben, eingesetzt.

Die Überführung der 2-Pyrazolin-4-essigsäuren der allgemeinen Formel I oder der Ausgestaltungen I* bzw. I** in ihre Salze erfolgt nach an sich bekannten Verfahren. Man erhält die Salze beispielsweise, indem man die 2-Pyrazolin-4-essigsäuren I mit dem stöchiometrischen Äquivalent an entsprechender Base umsetzt oder leicht lösliche Salze durch doppelte Umsetzung in schwer lösliche Salze umwandelt. Man erhält die Salze der 2-Pyrazolin-4-essigsäuren I aber auch, indem man die Umsetzung der Lactone II mit den Hydrazinen III in basischer Lösung durchführt und entsprechend aufarbeitet.

Die Ausgangsverbindungen II und III sind bekannt oder werden nach üblichen Verfahren aus bekannten Ausgangsmaterialien hergestellt.

Die 1,3-disubstituierten 2-Pyrazolin-4-essigsäuren der allgemeinen Formel I und ihre Salze bzw. die Ausgestaltungen I* und I** besitzen wertvolle Eigenschaften, die sie gewerblich verwertbar machen. Die erfindungsgemäßen Verbindungen zeigen eine ausgeprägte Fluoreszenz. Sie können zum optischen Aufhellen von synthetischen, halbsynthetischen oder natürlichen organischen Materialien verwendet werden. Ein weiterer Gegenstand der Erfindung ist daher die Verwendung der 1,3-disubstituierten 2-Pyrazolin-4-essigsäuren I, insbesondere die der Ausgestaltung I*, und ihrer Salze als optischer Aufheller oder Fluoreszenzfarbstoff.
Die 1,3-disubstituierten 2-Pyrazolin-4-essigsäuren der Ausgestaltung I** und ihre Salze sind darüber hinaus wertvolle Zwischenprodukte für die Herstellung von 1,3-disubstituierten Pyrazol-4-essigsäuren der allgemeinen Formel IV**

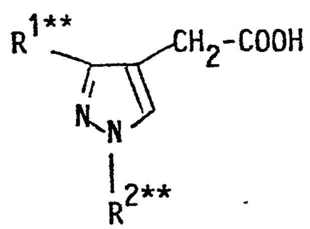

(IV**),

worin $R^{1**}$ und $R^{2**}$ die oben angegebene Bedeutung haben, und ihrer Salze. Ein weiterer Gegenstand der Erfindung ist daher die Verwendung der 2-Pyrazolin-4-essigsäuren I** und ihrer Salze bei der Herstellung von Pyrazol-4-essigsäuren der allgemeinen Formel IV** und ihren Salzen.

Die Verwendung der erfindungsgemäßen Verbindungen als optischer Aufheller oder Fluoreszenzfarbstoff erfolgt nach an sich bekannter Weise. Die Verbindungen können zum Färben von Fasern, Folien oder Geweben, z.B. aus Wolle, Seide, Cellulose, Celluloseestern, Polyamiden, Polyestern, Casein-Kunststoffen oder deren Gemischen benutzt werden. Die erfindungsgemäßen Verbindungen können den zu färbenden Materialien vor oder während deren Verarbeitung zugesetzt oder in sie eingearbeitet

werden. Die neuen Verbindungen können für sich allein, z.B. in Seifen oder Waschmitteln, zur Anwendung gelangen. Sie können aber auch mit anderen Farbstoffen, z.B. anderen optischen Aufhellern, verwendet werden, wobei sie diesen eine erhöhte Brillanz erteilen. Sie können auch zusammen mit verschiedenen Kunstharzen zur Herstellung von fluoreszierenden Pigmenten, Oberzügen etc. Anwendung finden. Dazu können sie in die zur Herstellung von Leuchtfarbstoffpigmenten üblichen Kunstharze, wie Kondensationsharze, z.B. Harnstoffformaldehydharze, Melaminformaldehydharze, oder Polymerisationsharze, z.B. Vinyl- oder Acrylharze, eingearbeitet werden. Ferner können die Verbindungen der Formel I auch bei der Herstellung von fluoreszierenden Erzeugnissen aus synthetischen Polykondensaten, z.B. gemäß dem Verfahren der französischen Patentschrift 1 155 365, eingesetzt werden, wobei den Verbindungen der Ausgestaltung I*, in denen $R^{4*}$ eine Carboxygruppe bedeutet, als Dicarbonsäuren eine besondere Bedeutung zukommt, da sie mit gutem Erfolg in die Polykondensatkette einpolymerisiert werden können.

Die Menge der erfindungsgemäß zu verwendenden neuen Verbindungen, bezogen auf das optisch aufzuhellende Material, kann in weiten Grenzen variieren. Bereits mit sehr geringen Mengen, z.B. solchen von 0,0001 Gewichtsprozent kann ein deutlicher Effekt erzielt werden. Es können aber auch Mengen bis zu 2 Gewichtsprozent zur Anwendung gelangen. Im allgemeinen hat sich ein Zusatz der erfindungsgemäßen Verbindungen von 0,0005 bis 0,5 Gewichtsprozent als zweckmäßig erwiesen.

Substituierte Pyrazol-4-essigsäuren sind als entzündungshemmende, analgetische und antipyretische Wirkstoffe bekannt. In der US-PS 4,146,721 und der GB-PS 1,373,212 werden Verfahren zu ihrer Herstellung, z.B. durch Lyolyse entsprechender Säurederivate oder Umsetzung von 1,3-Dicarbonylverbindungen mit Hydrazinen, beschrieben. Diese Verfahren sind wegen der damit verbundenen Stufen zur Herstellung der Ausgangsprodukte zeit- und arbeitsaufwendig, zum Teil auch abwasserbelastend. Die erfindungsgemäße Verwendung der 2-Pyrazolin-4-essigsäuren I** zur Herstellung von substituierten Pyrazol-4-essigsäuren IV** vermeidet nun die Nachteile der bisher bekannten Verfahren. Zur Herstellung von Pyrazol-4-essigsäuren IV** und ihrer Salze werden die 2-Pyrazolin-4-essigsäuren I** oder ihre Salze nach an sich bekannten Methoden oxydiert.

Die Oxydation erfolgt durch Umsetzung mit einem Dehydrierungs- oder Oxydationsmittel. Als solche kommen Metallkatalysatoren, wie Platin oder Palladium, die bei erhöhter Temperatur eine Wasserstoffabspaltung bewirken, ferner Wasserstoffakzeptoren, wie Tetrachloro-p-benzochinon, oder Oxydationsmittel, wie Sauerstoff, aktiviertes Mangandioxid, Kaliumpermanganat, Brom oder Wasserstoffperoxid, die in mindestens molaren Mengen eingesetzt werden, in Frage. Die Reaktion wird in einem inerten Lösungsmittel, beispielsweise Xylol, Toluol, Dichlorethan, Chloroform, Methylenchlorid, Aceton, Essigsäure oder Alkalilaugen (wie Natron- oder Kalilauge) bei Temperaturen von Raumtemperatur bis Siedetemperatur, vorzugsweise der Rückflußtemperatur, des Lösungsmittels durchgeführt, wobei die Bedingungen vom eingesetzten Dehydrierungs- oder Oxydationsmittel abhängen. Als Oxydationsmittel seien beispielsmäßig erwähnt Mangandioxid in Aceton, Essigsäure oder Dichlorethan; Luft in Gegenwart von Pd/C oder Pt/C in Natronlauge oder Kalilauge oder Xylol; Wasserstoffperoxid in Kalilauge; Wasserstoffperoxid/Brom in Kalilauge; vorzugsweise Kaliumpermanganat in Natronlauge oder Kalilauge und Mangandioxid in Dichlorethan.

Die Oberführung der Lactone II** in die Pyrazol-4-essigsäuren IV** im technischen oder halbtechnischen Maßstab kann alternativ ohne Isolierung der 2-Pyrazolin-4-essigsäuren I** als Eintopfreaktion durchgeführt werden. Beispielsweise werden die Lactone II** mit den Hydrazinen III** in einem entsprechenden Reaktionsgefäß umgesetzt, die Reaktionslösung mit einem geeigneten Lösungsmittel, wie Dichlorethan oder Chloroform extrahiert, die Reaktionslösung abgelassen und dann die Extraktionsphase mit dem Oxydationsmittel versetzt. Alternativ wird die 2-Pyrazolin-4-essigsäure I** durch Zugabe von Wasser gefällt, filtriert bzw. die überstehende Lösung dekantiert, der Rückstand in wäßriger Natronlauge oder Kalilauge aufgenommen und mit dem Oxydationsmittel, z.B. Kaliumpermanganat, umgesetzt.

Die Oberführung der Pyrazol-4-essigsäuren der allgemeinen Formel IV** in ihre Salze erfolgt nach an sich bekannten Verfahren. Man erhält die Salze beispielsweise, in dem man die Pyrazol-4-essigsäuren IV** mit dem

stöchiometrischen Äquivalent an entsprechender Base umsetzt oder leicht lösliche Salze durch doppelte Umsetzung in schwer lösliche Salze umwandelt oder beliebige Salze in pharmakologisch verträgliche Salze überführt. Man erhält die Salze der Pyrazol-4-essigsäuren IV** aber auch, indem man die Oxydation der Pyrazoline I** in basischer Lösung durchführt und entsprechend aufarbeitet.

Die folgenden Beispiele erläutern die Erfindung näher, ohne sie einzuschränken. Die angeführten Temperaturen sind in $^{o}C$ angegeben. Die Abkürzung "F." bedeutet Schmelzpunkt, die Abkürzung "Kp." Siedepunkt, die Abkürzung "Zers." Zersetzung.

**Beispiel 1**

**3-(4-Chlorphenyl)-1-(4-methoxyphenyl)-2-pyrazolin-4-essigsäure**

102 g 3-(4-Chlorbenzoyl)-4-hydroxybuttersäurelacton, 87 g 4-Methoxyphenylhydrazin-hydrochlorid und 177 g Natriumacetat · $3H_2O$ werden unter Stickstoffbegasung 50 Stunden in 1 l 50 %iger wässeriger Essigsäure unter Rückfluß zum Sieden erhitzt. Die Reaktionsmischung wird noch heiß mit 1 l Wasser versetzt, abgekühlt und 4 mal mit je 300 ml Methylenchlorid extrahiert; die vereinigten organischen Phasen werden 4 mal mit je 150 ml 2n Salzsäure und 2 mal mit Wasser gewaschen. Anschließend extrahiert man die organische Phase 5 mal mit je 500 ml 0,5 n Natronlauge, stumpft die vereinigten wässerigen Phasen mit 1n Salzsäure auf pH 9-10 ab, klärt mit Aktivkohle, filtriert und fällt unter Rühren mit 1n Salzsäure bis pH 3. Man erhält 113 g 3-(4-Chlorphenyl)-1-(4-methoxyphenyl)-2-pyrazolin-4-essigsäure (72 % d.Th.) als grüngelben Feststoff vom F. 120-122$^{\circ}$. Chromatographie an Kieselgel mit Chloroform/Methanol (19:1) liefert einen $R_f$-Wert von 0,65.

**Beispiel 2**

**3-(4-Chlorphenyl)-1-(4-methoxyphenyl)-pyrazol-4-essigsäure**

a) 6,9 g 3-(4-Chlorphenyl)-1-(4-methoxyphenyl)-2-pyrazolin-4-essigsäure werden in 70 ml Wasser unter Zusatz von 2,25 g Kaliumhydroxid gelöst; bei 50$^{\circ}$ wird eine Lösung von 4 g Kaliumpermanganat in 30 ml Wasser zugetropft und anschließend wird 2 Stunden unter Sieden zum Rückfluß erwärmt. Man setzt 0,5 g Aktivkohle zu, rührt 10 Minuten unter Rückfluß, läßt auf 40$^{\circ}$ abkühlen, filtriert und gibt bis zur Entfärbung der Lösung Natriumhydrogensulfit zu. Anschließend fällt man mit 1n Salzsäure bis pH 2. Man extrahiert 3 mal mit je 50 ml Methylenchlorid, wäscht die vereinigten organischen Phasen 2 mal mit 20 ml Wasser, konzentriert die organische Phase auf 50 ml, versetzt mit 30 ml Toluol und destilliert das restliche Methylenchlorid vollständig ab. Man erhält 6 g der Titelverbindung (87 % d.Th.), F. 140-142$^{\circ}$.

b) Alternativ wird die Titelverbindung wie folgt erhalten:
Man behandelt 102 g 3-(4-Chlorbenzoyl)-4-hydroxybuttersäurelacton nach der in Beispiel 5 angegebenen Arbeitsweise mit 87 g 4-Methoxyphenylhydrazinhydrochlorid und 177 g Natriumacetat · $3H_2O$ in 1 l 60 %iger Essigsäure. Die Lösung des Zwischenproduktes 3-(4-Chlorphenyl)-1-(4-methoxyphenyl)-2-pyrazolin-4-essigsäure in 1,5 l 1,2-Dichlorethan wird mit 300 g aktiviertem Mangandioxid versetzt, 2 Stunden am Wasserabscheider unter Rückfluß zum Sieden erhitzt, filtriert und das Filtrat vollständig eingeengt. Das Destillat kann wieder verwendet werden. Der Rückstand wird in 1n Kalilauge bei pH 11 unter Erwärmen gelöst, 2 mal mit je 100 ml Methylenchlorid extrahiert, auf pH 9 abgestumpft, abermals mit 2 mal 100 ml Methylenchlorid extrahiert, mit Aktivkohle geklärt, filtriert und aus dem Filtrat das Produkt durch Zugabe von 1n Salzsäure bis pH 2 gefällt. Ausbeute 109 g (70 % d.Th.). Umkristallisation aus Ethanol/Wasser liefert 88 g 3-(4-Chlorphenyl)-1-(4-methoxyphenyl)-pyrazol-4-essigsäure vom F. 139-141$^{\circ}$.

Beispiel 3

3-(4-Chlorphenyl)-1-phenyl-2-pyrazolin-4-essigsäure

Nach der in Beispiel 1 beschriebenen Arbeitsweise erhält man aus 270 g 3-(4-Chlorbenzoyl)-4-hydroxybuttersäurelacton, 155 g Phenylhydrazin und 240 g Natriumacetat in 2 l 50 %iger Essigsäure 342 g 3-(4-Chlorphenyl)-1-phenyl-2-pyrazolin-4-essigsäure (90 % d.Th.) als gelben Feststoff vom F. 142-146$^{\circ}$. Zur Reinigung wird aus Toluol umkristallisiert. Ausbeute 290 g gelbes Kristallisat vom F. 144-146$^{\circ}$.

Beispiel 4

3-(4-Chlorphenyl)-1-phenyl-pyrazol-4-essigsäure

a) 500 g 3-(4-Chlorphenyl)-1-phenyl-2-pyrazolin-4-essigsäure werden in 10 l 1,2-Dichlorethan gelöst, mit 1 kg Mangandioxid versetzt und am Wasserabscheider 5 Stunden unter Rückfluß zum Sieden erhitzt. Anschließend wird das Lösungsmittel abdestilliert, der Rückstand mit 15 l Aceton aufgekocht und noch warm filtriert, das Filtrat weitgehend eingeengt und bis zur Trübung mit Wasser versetzt.

Man kühlt im Eisbad ab, filtriert und wäscht mit Wasser nach. Man erhält 450 g der Titelverbindung (90 % d.Th.) vom F. 147-148°.

Alternativ wird die Titelverbindung wie folgt erhalten:

b) Man behandelt 6,3 g 3-(4-Chlorphenyl)-1-phenyl-2-pyrazolin-4-essigsäure nach der in Beispiel 2a) angegebenen Arbeitsweise in wässeriger Kaliumhydroxidlösung (2,25 g Kaliumhydroxid) mit 4 g Kaliumpermanganat und fällt die Titelverbindung durch Zugabe von 1n Salzsäure bis pH 2 als farblosen Feststoff vom F. 146-148°. Ausbeute: 5,7 g (91 % d.Th.).

c) 3,15 g 3-(4-Chlorphenyl)-1-phenyl-2-pyrazolin-4-essigsäure werden in 50 ml 0,4 molarer Kalilauge gelöst, mit 0,3 g Platin auf Aktivkohle (5 % Pt) versetzt und auf Rückflußtemperatur aufgeheizt. Dann wird 10 Stunden Sauerstoff durch die Reaktionslösung geleitet. Filtration vom Katalysator und Fällen durch Zugabe von 1n Salzsäure bis pH 3 liefert 1,70 g der Titelverbindung. Sie ist nach DC, F. und NMR identisch mit den nach 4a) und 4b) erhaltenen Produkten.

d) 5 g 3-(4-Chlorphenyl)-1-phenyl-2-pyrazolin-4-essigsäure werden in 100 ml 1,2-Dichlorethan analog der in Beispiel 4a) angegebenen Weise mit 10 g Mangandioxid behandelt. Nach dem Abdestillieren des Dichlorethans wird der Rückstand mit 80 ml 0,5 n Kalilauge ausgekocht, heiß filtriert, auf pH 9 abgestumpft und nach Abkühlung auf 20° 3mal mit 20 ml Dichlormethan extrahiert. Anschließend klärt man die Wasserphase mit Aktivkohle bei 90°, filtriert und fällt durch Zugabe von 1 n Salzsäure bis pH 2.
Man erhält 4,2 g der Titelverbindung vom F. 147-148°.

Beispiel 5

3-(3-Chlorphenyl)-1-phenyl-pyrazol-4-essigsäure

4,5 g 3-(3-Chlorbenzoyl)-4-hydroxybuttersäurelacton, 2,8 g Phenylhydrazin und 4 g Natriumacetat werden in 30 ml 50 %iger Essigsäure 50 Stunden unter Stickstoffbegasung zum Sieden erhitzt, mit 30 ml Wasser verdünnt und nach Abkühlung auf Raumtemperatur 4 mal mit 40 ml 1,2-Dichlorethan extrahiert. Die vereinigten, 3-(3-Chlorphenyl)-1-phenyl-2-pyrazolin-4-essigsäure enthaltenden organischen Phasen werden 3 mal mit je 20 ml 0,5 n Salzsäure und anschließend 2 mal mit 20 ml gesättigter Natriumchloridlösung gewaschen. Danach versetzt man die organische Phase mit 15 g Mangandioxid und erhitzt 3 Stunden am Wasserabscheider zum Sieden. Man filtriert die noch warme Lösung und engt das Filtrat vollständig ein. Das zurückgewonnene Lösungsmittel kann erneut verwendet werden. Der Filterkuchen wird mit 100 ml Aceton ausgekocht, abermals abfiltriert und das Filtrat mit dem aus der ersten Filtration gewonnenen Filtrat vereinigt. Das Lösungsmittel wird zur Trockne eingeengt und der Rückstand aus Toluol umkristallisiert. Man erhält 5,20 g der Titelverbindung vom F. 122-124$^{o}$.

Beispiel 6

3-(4-Methylphenyl)-1-phenyl-pyrazol-4-essigsäure

Nach der in Beispiel 5 beschriebenen Arbeitsweise erhält man durch Umsetzung von 4,38 g 3-(4-Methylbenzoyl)-4-hydroxybuttersäurelacton, 2,65 g Phenylhydrazin und 3 g Natriumacetat in 40 ml 70 %iger Essigsäure und nachfolgende Oxidation des Zwischenproduktes 3-(4-Methylphenyl)-1-phenyl-2-pyrazolin-4-essigsäure mit 15 g Mangandioxid 4,5 g der Titelverbindung vom F. 139-141$^{o}$.

Beispiel 7

3-(3,4-Dichlorphenyl)-1-phenyl-pyrazol-4-essigsäure

Nach der in Beispiel 5 beschriebenen Arbeitsweise erhält man durch Umsetzung von 2,6 g 3-(3,4-Dichlorbenzoyl)-4-hydroxybuttersäurelacton, 3,0 g Phenylhydrazin und 3 g Natriumacetat und Oxidation des Zwischenproduktes 3-(3,4-Dichlorphenyl)-1-phenyl-2-pyrazolin-4-essigsäure mit 8 g Mangandioxid 2,6 g der Titelverbindung vom F. 170-172$^{o}$.

0094555

Beispiel 8

1-(4-Fluorphenyl)-3-phenyl-pyrazol-4-essigsäure

Nach der in Beispiel 5 beschriebenen Arbeitsweise erhält man durch Umsetzung von 5,67 g 3-Benzoyl-4-hydroxybuttersäurelacton, 4,5 g 4-Fluorphenylhydrazin und 10 g Natriumacetat in 100 ml 50 %iger Essigsäure nach Verdünnen mit Wasser 1-(4-Fluorphenyl)-3-phenyl-2-pyrazolin-4-essigsäure als amorphes gelbes Produkt, das nach Dekantieren der überstehenden Lösung in 50 ml 2n Kaliumhydroxidlösung mit 5 g Kaliumpermanganat nach der in Beispiel 2 a) beschriebenen Arbeitsweise oxidiert wird. Man erhält 7,1 g (80 % d.Th.) der Titelverbindung vom F. 124-126°.

Beispiel 9

1,3-Bis-(4-chlorphenyl)-2-pyrazolin-4-essigsäure

9 g 3-(4-Chlorbenzoyl)-4-hydroxybuttersäurelacton, 12 g 4-Chlorphenylhydrazinsulfat und 10 g Pyridin werden unter Stickstoffbegasung in 200 ml Eisessig 60 Stunden bei 95° gerührt und nach der in Beispiel 1 angegebenen Arbeitsweise aufgearbeitet. Man erhält 7 g (54 % d.Th.) 1,3-Bis-(4-chlorphenyl)-2-pyrazolin-4-essigsäure vom F. 175-177°.

Beispiel 10

1,3-Bis-(4-chlorphenyl)-pyrazol-4-essigsäure

6,98 g 1,3-Bis-(4-chlorphenyl)-2-pyrazolin-4-essigsäure und 15 g Mangandioxid werden in 70 ml Eisessig 5 Stunden unter Rückfluß zum Sieden erhitzt; es wird heiß filtriert, der Filterkuchen wird mit 100 ml Aceton ausgekocht; die beiden Filtrate werden vereinigt und auf 20 ml eingeengt, das Produkt wird mit Wasser zur Kristallisation gebracht. Man erhält 5,1 g der Titelverbindung vom F. 195-196°. Chromatographie an Kieselgel mit Chloroform/Methanol (9:1) liefert einen $R_f$-Wert von 0,30.

Beispiel 11

3-(4-Fluorphenyl)-1-phenyl-pyrazol-4-essigsäure

Nach der in Beispiel 5 beschriebenen Arbeitsweise erhält man durch Umsetzung von 4 g 3-(4-Fluorbenzoyl)-4-hydroxybuttersäurelacton, 2,8 g Phenylhydrazin, 4 g Natriumacetat und anschließende Oxidation des Zwischenproduktes 3-(4-Fluorphenyl)-1-phenyl-2-pyrazolin-4-essigsäure mit 15 g Mangandioxid 4,8 g der Titelverbindung vom F. 149-151°.

Beispiel 12

3-(4-Bromphenyl)-1-phenyl-pyrazol-4-essigsäure

Nach der in Beispiel 5 beschriebenen Arbeitsweise erhält man durch Umsetzung von 5,40 g 3-(4-Brombenzoyl)-4-hydroxybuttersäurelacton, 3,0 g Phenylhydrazin und 6 g Natriumacetat und anschließende Oxidation des Zwischenproduktes 3-(4-Bromphenyl)-1-phenyl-2-pyrazolin-4-essigsäure mit 18 g Mangandioxid 5,5 g der Titelverbindung vom F. 140-142°.

Beispiel 13

1-Phenyl-3-(2-thienyl)-pyrazol-4-essigsäure

Nach der in Beispiel 5 beschriebenen Arbeitsweise erhält man durch Umsetzung von 3,8 g 3-(2-Thenoyl)-4-hydroxybuttersäurelacton, 2,8 g Phenylhydrazin und 4 g Natriumacetat und nachfolgende Oxidation des Zwischenproduktes 1-Phenyl-3-(2-thienyl)-2-pyrazolin-4-essigsäure mit 15 g Mangandioxid 4,8 g der Titelverbindung. Chromatographie an Kieselgel mit Toluol/Cyclohexan/Eisessig (6:3:3) liefert einen $R_f$-Wert von ca. 0,6.

Beispiel 14

3-(2-Naphthyl)-1-phenyl-pyrazol-4-essigsäure

Nach der in Beispiel 5 beschriebenen Arbeitsweise erhält man durch Umsetzung von 5,5 g 3-(2-Naphthoyl)-4-hydroxybuttersäurelacton, 2,5 g Phenylhydrazin und 4 g Natriumacetat nach Verdünnen mit Wasser 3-(2-Naphthyl)-1-phenyl-2-pyrazolin-4-essigsäure als blaßgelbes Zwischenprodukt, das bei der Oxidation mit 5 g Kaliumpermanganat nach der in Beispiel 2 a) beschriebenen Arbeitsweise zur Titelverbindung oxidiert wird. Ausbeute 5,3 g (81 % d.Th.) vom F. 175-176°.

Beispiel 15

1,3-Diphenyl-2-pyrazolin-4-essigsäure

7,6 g 3-Benzoyl-4-hydroxybuttersäurelacton und 5,5 g Phenylhydrazin werden unter Stickstoffbegasung in 200 ml Dioxan und 200 ml eines 0,5 normalen Zitronensäurepuffergemisches von pH 4,5 70 Stunden zum Sieden erhitzt. Aufarbeitung nach der in Beispiel 1 angegebenen Arbeitsweise liefert 8,2 g (73 % d.Th.) der Titelverbindung vom F. 150-152°.

Beispiel 16

1,3-Diphenyl-pyrazol-4-essigsäure

Nach der in Beispiel 4a) beschriebenen Arbeitsweise erhält man aus 3 g 1,3-Diphenyl-2-pyrazolin-4-essigsäure und 8 g Mangandioxid in 60 ml 1,2-Dichlorethan 2,2 g (73 % d.Th.) der Titelverbindung vom F. 109-111°.

Beispiel 17

3-(4-Chlorphenyl)-1-(2-pyridyl)-2-pyrazolin-4-essigsäure

8 g 3-(4-Chlorbenzoyl)-4-hydroxybuttersäurelacton, 6 g 2-Hydrazinopyridin und 6 g Natriumacetat werden unter Stickstoffbegasung in 80 ml 50 %iger Essigsäure 60 Stunden unter Rückfluß zum Sieden erhitzt. Nach Zugabe von 40 ml Wasser wird abgekühlt, der Feststoff abgesaugt, mit einer Pufferlösung aus Essigsäure und Natriumacetat von pH 5 und anschließend mit 100 ml Wasser gewaschen, 1 mal mit 50 ml Diethylether ausgerührt und getrocknet. Man erhält 10,5 g (93 % d.Th.) 3-(4-Chlorphenyl)-1-(2-pyridyl)-2-pyrazolin-4-essigsäure vom F. 224-225°.

Beispiel 18

3-(4-Chlorphenyl)-1-(2-pyridyl)-pyrazol-4-essigsäure

Nach der in Beispiel 4 a) beschriebenen Arbeitsweise erhält man aus 6,35 g 3-(4-Chlorphenyl)-1-(2-pyridyl)-2-pyrazolin-4-essigsäure und 20 g Braunstein in 400 ml Dichlorethan 5,1 g (81 % d.Th.) der Titelverbindung vom F.164-166°. Chromatographie an Kieselgel mit Chloroform/Methanol (19:1) liefert einen $R_f$-Wert von 0,50.

0094555

Beispiel 19

1-Phenyl-3-(3-pyridyl)-2-pyrazolin-4-essigsäure

Nach der in Beispiel 17 beschriebenen Arbeitsweise erhält man aus 3,82 g 3-(3-Pyridoyl)-4-hydroxybuttersäurelacton, 3 g Phenylhydrazin und 5 g Natriumacetat in 50 ml 40 %iger Essigsäure 3,0 g 1-Phenyl-3-(3-pyridyl)-2-pyrazolin-4-essigsäure, die ohne Reinigung weiter verarbeitet werden. Chromatographie an Kieselgel mit Benzol/Cyclohexan/Eisessig (6:3:3) liefert einen $R_f$-Wert von ca. 0,1.

Beispiel 20

1-Phenyl-3-(3-pyridyl)-pyrazol-4-essigsäure

Nach der in Beispiel 4 a) beschriebenen Arbeitsweise erhält man aus 5,60 g 1-Phenyl-3-(3-pyridyl)-2-pyrazolin-4-essigsäure und 16 g Braunstein in 200 ml Eisessig 4,70 g (84 % d.Th.) der Titelverbindung vom F. 216-218$^{0}$.

Beispiel 21

3-(4-Chlorphenyl)-1-(3-methylphenyl)-pyrazol-4-essigsäure

Nach der in Beispiel 5 beschriebenen Arbeitsweise erhält man durch Umsetzung von 4,5 g 3-(4-Chlorbenzoyl)-4-hydroxybuttersäurelacton, 3,0 g 3-Methylphenylhydrazin und 4 g Natriumacetat in 40 ml 50 %iger Essigsäure und nachfolgende Oxidation des Zwischenproduktes 3-(4-Chlorphenyl)-1-(3-methylphenyl)-2-pyrazolin-4-essigsäure mit 15 g Braunstein 5,5 g der Titelverbindung vom F. 128-130$^{0}$.

Beispiel 22

3-(4-Chlorphenyl)-1-phenyl-2-pyrazolin-4-essigsäure-Calcium-Salz

3 g (9,5 mMol) 3-(4-Chlorphenyl)-1-phenyl-2-pyrazolin-4-essigsäure werden in 10 ml Kalilauge unter Erwärmung gelöst, auf 100 ml verdünnt und auf 90$^{0}$ erwärmt. In die heiße Lösung werden unter Rühren 22 ml 5 %ige Calciumchloridlösung eingetropft. Der gelbe Feststoff wird nach Abkühlen auf 20$^{0}$ filtriert, mit Wasser chloridfrei gewaschen und getrocknet. Man erhält 3,2 g (95 % d.Th.) der Titelverbindung. Zersetzungspunkt > 330$^{0}$.

Beispiel 23

3-(4-Chlorphenyl)-1-phenylpyrazol-4-essigsäure-Calcium-Salz

50 g 3-(4-Chlorphenyl)-1-phenyl-2-pyrazolin-4-essigsäure werden nach der in Beispiel 2 a) angegebenen Arbeitsweise mit 18 g Kaliumhydroxid und 32 g Kaliumpermanganat in 500 ml Wasser behandelt und bis zur Zugabe des Natriumhydrogensulfits analog Beispiel 2 a) aufgearbeitet. Anschließend erwärmt man auf 80° und fällt das Produkt durch Zugabe von 9 g Calciumchlorid, gelöst in 180 ml Wasser. Man rührt 1 Stunde nach, kühlt auf Raumtemperatur, filtriert, wäscht mit Wasser chloridfrei und trocknet. Man erhält 46 g (88 % d.Th.) der Titelverbindung vom F. 260-280°.

Beispiel 24

1-(4-Carboxyphenyl)-3-(4-chlorphenyl)-2-pyrazolin-4-essigsäure

4,5 g 3-(4-Chlorbenzoyl)-4-hydroxybuttersäurelacton, 3,65 g 4-Hydrazinobenzoesäure und 3,4 g Natriumacetat werden in 50 ml 50 %iger Essigsäure 5 Stunden unter Rückfluß zum Sieden erhitzt, mit 50 ml Wasser versetzt und nach Abkühlung der Feststoff filtriert. Zur Reinigung wird das Produkt in 1 n Kalilauge bei pH 9 gelöst, 3 mal mit je 20 ml Dichlormethan extrahiert, mit Aktivkohle geklärt, filtriert und durch Zugabe von 1 n Salzsäure bis pH 2 gefällt. Nach Umkristallisation aus Aceton/Wasser erhält man 3,8 g der Titelverbindung; Zers. ab 255°.

Beispiel 25

3-(4-Chlorphenyl)-1-(4-sulfophenyl)-2-pyrazolin-4-essigsäure

Nach der in Beispiel 24 beschriebenen Arbeitsweise erhält man durch Umsetzung von 4,5 g 3-(4-Chlorbenzoyl)-4-hydroxybuttersäurelacton, 4,73 g 4-Hydrazinophenylsulfonsäurehemihydrat und 7,93 g Natriumacetat • $3H_2O$ in 50 ml 50 %iger Essigsäure 5,0 g (63 % d.Th.) der Titelverbindung vom F. 210°.

Beispiel 26

1-(2-Benzthiazolyl)-3-(4-chlorphenyl)-2-pyrazolin-4-essigsäure

Nach der in Beispiel 17 beschriebenen Arbeitsweise erhält man durch Umsetzung von 4,5 g 3-(4-Chlorbenzoyl)-4-hydroxybuttersäurelacton, 3,3 g 2-Hydrazinobenzthiazol und 3,4 g Natriumacetat 6,3 g eines gelben Feststoffes. Nach Ausrühren mit 30 ml heißem Methanol erhält man 5,9 g (78 % d.Th.) der Titelverbindung vom F. 316-318°. Chromatographie an Kieselgel mit Chloroform/Methanol liefert einen $R_f$-Wert von 0,55.

Beispiel 27

10 kg eines 60 % Gesamtfettsäure enthaltenden Seifensuds werden 1,0 g 1-(4-Carboxyphenyl)-3-(4-chlorphenyl)-2-pyrazolin-4-essigsäure, in Form einer Suspension in Seifenlösung, zugesetzt. Die Seife ist gut aufgehellt.

Beispiel 28

Gewebe aus Rohwolle, die etwas vergilbt aussieht, wird bei 40-50° im Flottenverhältnis 1:20 20 Minuten mit einer Bleichlösung behandelt, die pro Liter 2 g Natriumhydrosulfit, 1 g Tetranatriumpyrophosphat, 0,5 g eines Netzmittels (Avolan®P) und 0,02 g 3-(4-Chlorphenyl)-1-(4-sulfophenyl)-2-pyrazolin-4-essigsäure enthält. Nach dem Spülen und Trocknen zeigt das Gewebe ein besseres Weiß als das, das mit der gleichen Bleichlösung ohne Zusatz der Pyrazolinverbindung behandelt wurde.

Beispiel 29

In einem Bad, das pro Liter 0,5 g des Natriumsalzes von 1-(2-Benzthiazolyl)-3-(4-chlorphenyl)-2-pyrazolin-4-essigsäure, 10 ml 2n Schwefelsäure und 3 g Natriumsulfat enthält, wird vergilbte Rohwolle (50 g) bei einem Flottenverhältnis von 1:50 30 Minuten bei 80-90° behandelt. Anschliessend wird das Material gespült und getrocknet. Man erhält ein deutlich aufgehelltes Material, das bei Bestrahlung mit Licht der Wellenlänge 366 nm kräftig blau fluoresziert.

Beispiel 30

Behandelt man Wolle unter denselben Bedingungen und mit denselben Zusätzen wie in Beispiel 29 mit 1-(4-Carboxyphenyl)-3-(4-chlorphenyl)-2-pyrazolin-4-essigsäure anstelle von 1-(2-Benzthiazolyl)-3-(4-chlorphenyl)-2-pyrazolin-4-essigsäure, so erhält man ein intensiv zitronengelb gefärbtes Material, das bei Bestrahlung mit Licht der Wellenlänge 366 nm kräftig blau fluoresziert.

Tabelle I gibt die Absorptions- und Fluoreszenzmaxima von erfindungsgemäßen 2-Pyrazolin-4-essigsäuren in methanolischer Lösung wieder.

Tabelle I

| Beispiel | Absorptionsmaximum (Methanol) λ [nm] | log ε | Fluoreszenzmaximum (Methanol) λ [nm] |
|---|---|---|---|
| 3 | 360 | 4,32 | 468 |
| 17 | 346 | 4,41 | 437 |
| 24 | 370 | 4,60 | 445 |
| 25 | 361 | 4,37 | 455 |
| 26 | 348 | 4,48 | 452 |

Die Ermittlung der Absorptionsmaxima erfolgte mit einem UV-Spektrometer Perkin-Elmer 555, die Ermittlung der Fluoreszenzmaxima erfolgte mit einem Fluorimeter Farrand MK1.

## Herstellung der Ausgangsprodukte

### Beispiel 31

3-(4-Chlorbenzoyl)-4-hydroxybuttersäurelacton

140 g 4-(4-Chlorphenyl)-4-oxo-buttersäure werden in 1450 ml 0,5 n Natronlauge gelöst und mit 57 ml 35 %iger wäßriger Formalinlösung versetzt. Nach zweistündigem Stehen bei $20^{0}$ wird mit halbkonzentrierter Salzsäure auf pH 2 angesäuert und anschließend unter kräftigem Rühren 5 Stunden zum Rückfluß erhitzt. Nach Abkühlung wird zweimal mit je 300 ml Methylenchlorid extrahiert, und anschließend werden die vereinigten organischen Phasen weitgehend eingeengt. Nach Zugabe von 200 ml gesättigter Natriumhydrogencarbonatlösung wird das restliche Methylenchlorid unter Rühren abdestilliert und anschließend im Eisbad gekühlt. Der ausgefallene Feststoff wird filtriert, mit Wasser gewaschen und getrocknet. Man erhält 115 g (78 % d.Th.) der Titelverbindung vom F. $82\text{-}83^{0}$.

### Beispiel 32

3-(3-Chlorbenzoyl)-4-hydroxybuttersäurelacton

Nach der in Beispiel 31 beschriebenen Arbeitsweise erhält man aus 14 g 4-(3-Chlorphenyl)-4-oxo-buttersäure, 5,7 ml 35 %iger Formalinlösung und 170 ml 0,5 n Natronlauge 10 g der Titelverbindung.

### Beispiel 33

3-(3,4-Dichlorbenzoyl)-4-hydroxybuttersäurelacton

Nach der in Beispiel 31 beschriebenen Arbeitsweise erhält man aus 6,2 g 4-(3,4-Dichlorphenyl)-4-oxo-buttersäure, 2,2 ml 35 %igem Formalin und 28 ml 1 n Natronlauge 4,5 g der Titelverbindung.

### Beispiel 34

3-(4-Fluorbenzoyl)-4-hydroxybuttersäurelacton

Nach der in Beispiel 31 beschriebenen Arbeitsweise erhält man aus 9,8 g 4-(4-Fluorphenyl)-4-oxo-buttersäure, 4,5 ml 35 %iger Formalinlösung und 115 ml 0,5 n Natronlauge 7,5 g der Titelverbindung.

Beispiel 35

3-(2-Thenoyl)-4-hydroxybuttersäurelacton

Nach der in Beispiel 31 beschriebenen Arbeitsweise erhält man aus 18,4 g 4-(2-Thienyl)-4-oxo-buttersäure, 9 ml 35 %iger Formalinlösung und 225 ml 0,5 n Natronlauge 15,5 g der Titelverbindung.

Beispiel 36

3-(2-Naphthoyl)-4-hydroxybuttersäurelacton

Nach der in Beispiel 31 beschriebenen Arbeitsweise erhält man aus 11,45 g 4-(2-Naphthyl)-4-oxo-buttersäure, 4,5 ml 35 %iger Formalinlösung und 115 ml 0,5 n Natronlauge 8,2 g der Titelverbindung.

Beispiel 37

3-(3-Pyridincarbonyl)-4-hydroxybuttersäurelacton

Nach der in Beispiel 31 beschriebenen Arbeitsweise erhält man aus 9 g 4-(3-Pyridyl)-4-oxo-buttersäure, 4,5 ml 35 %iger Formalinlösung und 110 ml 0,5 n Natronlauge 6,4 g der Titelverbindung.

Patentansprüche (DE,FR,IT,NL,BE,CH,GB,SE,LU)

1. 1,3-Disubstituierte 2-Pyrazolin-4-essigsäuren der allgemeinen Formel I

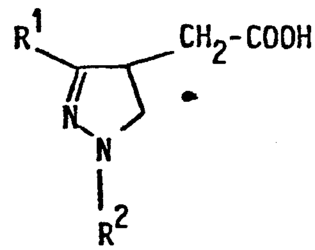

(I),

worin

$R^1$ eine gegebenenfalls substituierte Arylgruppe oder eine gegebenenfalls substituierte Heteroarylgruppe und

$R^2$ eine gegebenenfalls substituierte Arylgruppe oder eine gegebenenfalls substituierte Heteroarylgruppe bedeuten,

und ihre Salze mit anorganischen oder organischen Basen.

2. 1,3-Disubstituierte 2-Pyrazolin-4-essigsäuren nach Anspruch 1, gekennzeichnet durch die allgemeine Formel I*

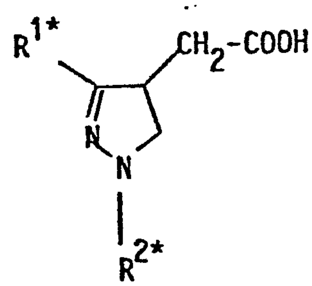

(I*),

worin

$R^{1*}$ einen Phenylrest, einen durch $R^{3*}$ substituierten Phenylrest, einen 4-Biphenylyl-Rest oder einen 2-Naphthylrest,

$R^{2*}$ einen durch $R^{4*}$ substituierten Phenylrest oder einen Benzthiazolylrest,

$R^{3*}$ ein oder zwei in 3- und/oder 4-Stellung ständige Halogenatome und

$R^{4*}$ eine in 4-Stellung ständige Carboxy-, Sulfo-, Aminosulfonyl-, Dimethylaminoniederalkylaminosulfonyl-, Niederalkylsulfonyl- oder eine ω-Sulfoniederalkylsulfonylgruppe bedeuten,

und ihre Salze.

3. Verbindungen der Ausgestaltung I* nach Anspruch 2, in denen $R^{1*}$ einen Phenylrest oder einen durch $R^{3*}$ substituierten Phenylrest, $R^{2*}$ einen durch $R^{4*}$ substituierten Phenylrest, $R^{3*}$ ein in 4-Stellung ständiges Chloratom oder zwei in 3- und 4-Stellung ständige Chloratome und $R^{4*}$ eine Carboxy-, eine Sulfo-, eine Aminosulfonyl-, eine Methylsulfonyl- oder eine 2-Sulfoethylsulfonylgruppe bedeuten.

4. 1,3-Disubstituierte 2-Pyrazolin-4-essigsäuren nach Anspruch 1, gekennzeichnet durch die allgemeine Formel I**

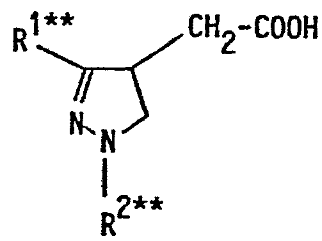

(I**),

worin

$R^{1**}$ einen Phenylrest, einen durch $R^{5**}$ substituierten Phenylrest, einen 4-Biphenylylrest, einen 2-Naphthylrest, einen 2-Thienylrest, einen 2-Furyl- oder einen 2- oder 3-Pyridylrest,

$R^{2**}$ einen Phenylrest, einen 2-Thienylrest, einen 2-Furyl- oder einen 2- oder 3-Pyridylrest,

$R^{5**}$ ein in 3- oder 4-Stellung ständiges Chlor- oder Fluoratom, eine in 3- oder 4-Stellung ständige Methyl- oder Methoxygruppe oder eine in 3-Stellung ständige Trifluormethylgruppe bedeuten,

und ihre Salze.

5. Verbindungen der Ausgestaltung I** nach Anspruch 4, in denen $R^{1**}$ einen Phenylrest, einen durch $R^{5**}$ substituierten Phenylrest oder einen 2-Thienylrest, $R^{2**}$ einen Phenylrest oder einen 2-Thienylrest und $R^{5**}$ ein in 4-Stellung ständiges Chloratom bedeuten.

6. Verbindung der Ausgestaltung I** nach Anspruch 4, in der $R^{1**}$ einen 4-Chlorphenylrest und $R^{2**}$ einen Phenylrest bedeuten.

7. Verfahren zur Herstellung von 2-Pyrazolin-4-essigsäuren der allgemeinen Formel I und ihren Salzen, dadurch gekennzeichnet, daß man ein Lacton der allgemeinen Formel II

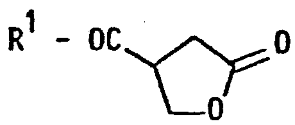 (II),

worin $R^1$ die oben angegebene Bedeutung hat, mit einem Hydrazin der allgemeinen Formel III

$$R^2 - NH - NH_2 \qquad (III),$$

worin $R^2$ die oben angegebene Bedeutung hat, umsetzt und gegebenenfalls anschließend die erhaltene Säure der allgemeinen Formel I in ein Salz oder ein erhaltenes Salz in die freie Säure überführt.

8. Verwendung von 2-Pyrazolin-4-essigsäuren der allgemeinen Formel I nach Anspruch 1 oder ihrer Salze als optischer Aufheller.

9. Verwendung von 2-Pyrazolin-4-essigsäuren der allgemeinen Formel I nach Anspruch 1 oder ihrer Salze als Fluoreszenzfarbstoff.

10. Verwendung von 2-Pyrazolin-4-essigsäuren der allgemeinen Formel I** oder ihrer Salze zur Herstellung von 1,3-disubstituierten Pyrazol-4-essigsäuren der allgemeinen Formel IV**

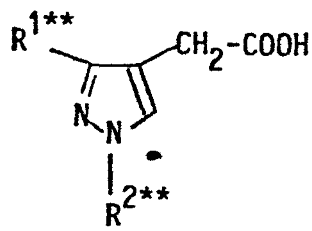

(IV**),

worin $R^{1**}$ und $R^{2**}$ die in Anspruch 4 angegebene Bedeutung haben, und ihrer Salze.

## Patentansprüche (Vertragsstaat AT)

1. Verfahren zur Herstellung von 1,3-disubstituierten 2-Pyrazolin-4-essigsäuren der allgemeinen Formel I

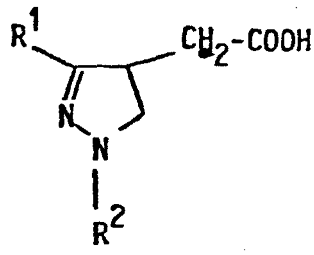

(I),

worin

$R^1$ eine gegebenenfalls substituierte Arylgruppe oder eine gegebenenfalls substituierte Heteroarylgruppe und

$R^2$ eine gegebenenfalls substituierte Arylgruppe oder eine gegebenenfalls substituierte Heteroarylgruppe bedeuten,

und ihren Salzen, dadurch gekennzeichnet, daß man ein Lacton der allgemeinen Formel II

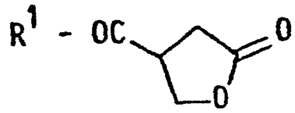

(II),

worin $R^1$ die oben angegebene Bedeutung hat, mit einem Hydrazin der allgemeinen Formel III

$$R^2 - NH - NH_2 \quad (III),$$

worin $R^2$ die oben angegebene Bedeutung hat, umsetzt und gegebenenfalls anschließend die erhaltene Säure der allgemeinen Formel I in ein Salz oder ein erhaltenes Salz in die freie Säure überführt.

2. Verfahren zur Herstellung von 1,3-disubstituierten 2-Pyrazolin-4-essigsäuren der allgemeinen Formel I*

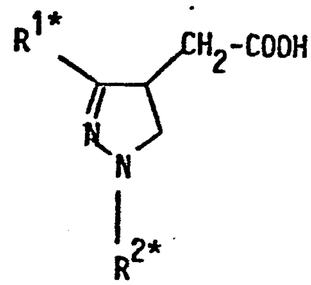

(I*),

worin
$R^{1*}$ einen Phenylrest, einen durch $R^{3*}$ substituierten Phenylrest, einen 4-Biphenylyl-Rest oder einen 2-Naphthylrest,
$R^{2*}$ einen durch $R^{4*}$ substituierten Phenylrest oder einen Benzthiazolylrest,
$R^{3*}$ ein oder zwei in 3- und/oder 4-Stellung ständige Halogenatome und
$R^{4*}$ eine in 4-Stellung ständige Carboxy-, Sulfo-, Aminosulfonyl-, Dimethylaminoniederalkylaminosulfonyl-, Niederalkylsulfonyl- oder eine ω-Sulfoniederalkylsulfonylgruppe bedeuten,
und ihren Salzen, dadurch gekennzeichnet, daß man ein Lacton der allgemeinen Formel II*

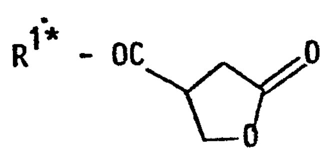

(II*),

worin $R^{1*}$ die oben angegebene Bedeutung hat, mit einem Hydrazin der allgemeinen Formel III*

$$R^{2*} - NH - NH_2 \quad (III^*),$$

worin $R^{2*}$ die oben angegebene Bedeutung hat, umsetzt und gegebenenfalls anschließend die erhaltene Säure der allgemeinen Formel I* in ein Salz oder ein erhaltenes Salz in die freie Säure überführt.

3. Verfahren nach Anspruch 2, dadurch gekennzeichnet, daß man ein Lacton der Formel II* und ein Hydrazin der Formel III* einsetzt, in denen $R^{1*}$ einen Phenylrest oder einen durch $R^{3*}$ substituierten Phenylrest, $R^{2*}$ einen durch $R^{4*}$ substituierten Phenylrest, $R^{3*}$ ein in 4-Stellung

ständiges Chloratom oder zwei in 3- und 4-Stellung ständige Chloratome und $R^{4*}$ eine Carboxy-, eine Sulfo-, eine Aminosulfonyl-, eine Methylsulfonyl- oder eine 2-Sulfoethylsulfonylgruppe bedeuten.

4. Verfahren zur Herstellung von 1,3-disubstituierten 2-Pyrazolin-4-essigsäuren der allgemeinen Formel I**

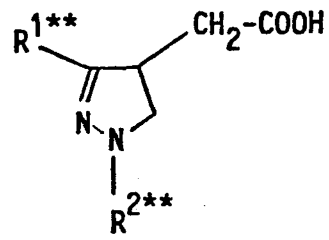

(I**),

worin

$R^{1**}$ einen Phenylrest, einen durch $R^{5**}$ substituierten Phenylrest, einen 4-Biphenylylrest, einen 2-Naphthylrest, einen 2-Thienylrest, einen 2-Furyl- oder einen 2- oder 3-Pyridylrest,

$R^{2**}$ einen Phenylrest, einen 2-Thienylrest, einen 2-Furyl- oder einen 2- oder 3-Pyridylrest,

$R^{5**}$ ein in 3- oder 4-Stellung ständiges Chlor- oder Fluoratom, eine in 3- oder 4-Stellung ständige Methyl- oder Methoxygruppe oder eine in 3-Stellung ständige Trifluormethylgruppe bedeuten,

und ihren Salzen, dadurch gekennzeichnet, daß man ein Lacton der allgemeinen Formel II**

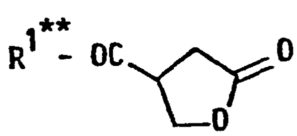

(II**)

worin $R^{1**}$ die oben angegebene Bedeutung hat, mit einem Hydrazin der allgemeinen Formel III**

$$R^{2**} - NH - NH_2 \qquad (III^{**}),$$

worin $R^{2**}$ die oben angegebene Bedeutung hat, umsetzt und gegebenenfalls anschließend die erhaltene Säure der allgemeinen Formel I** in ein Salz oder ein erhaltenes Salz in die freie Säure überführt.

5. Verfahren nach Anspruch 4, dadurch gekennzeichnet, daß man ein Lacton der Formel II** und ein Hydrazin der Formel III** einsetzt, in denen $R^{1**}$ einen Phenylrest, einen durch $R^{5**}$ substituierten Phenylrest oder einen 2-Thienylrest, $R^{2**}$ einen Phenylrest oder einen 2-Thienylrest und $R^{5**}$ ein in 4-Stellung ständiges Chloratom bedeuten.

6. Verfahren nach Anspruch 4, dadurch gekennzeichnet, daß man ein Lacton der Formel II** und ein Hydrazin der Formel III** einsetzt, in denen $R^{1**}$ einen 4-Chlorphenylrest und $R^{2**}$ einen Phenylrest bedeuten.

7. Verwendung von nach Anspruch 1 erhaltenen 2-Pyrazolin-4-essigsäuren der allgemeinen Formel I oder ihren Salzen als optischer Aufheller.

8. Verwendung von nach Anspruch 1 erhaltenen 2-Pyrazolin-4-essigsäuren der allgemeinen Formel I oder ihren Salzen als Fluoreszenzfarbstoff.

9. Verwendung von nach Anspruch 4 erhaltenen 2-Pyrazolin-4-essigsäuren der allgemeinen Formel I** oder ihren Salzen zur Herstellung von 1,3-disubstituierten Pyrazol-4-essigsäuren der allgemeinen Formel IV**

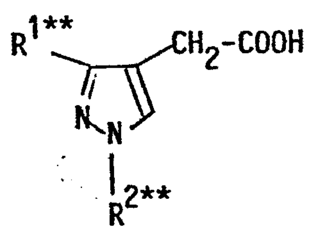

(IV**),

worin $R^{1**}$ und $R^{2**}$ die in Anspruch 4 angegebene Bedeutung haben, und ihren Salzen.

Europäisches Patentamt

# EUROPÄISCHER RECHERCHENBERICHT

0094555
Nummer der Anmeldung

EP 83 10 4373

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | CHEMICAL ABSTRACTS, Band 86, Nr. 13, 28. März 1977, Seite 532, Nr. 89682v, Columbus, Ohio, USA I.A. ALEKSANDROVA et al.: "Reactions of diazomethane with unsaturated compounds of the furan series" & ZH. ORG. KHIM. 1976, 12(11), 2433-6 * Zusammenfassung *<br>----- | 1 | C 07 D 231/06<br>C 07 D 409/04<br>C 07 D 401/04<br>C 07 D 417/04<br>C 07 D 231/12<br>C 07 D 307/32<br>C 07 D 409/06<br>C 07 D 405/06<br>A 61 K 31/415 |
| | | | RECHERCHIERTE SACHGEBIETE (Int. Cl. 3) |
| | | | C 07 D 231/00<br>C 07 D 409/00<br>C 07 D 401/00 |

Der vorliegende Recherchenbericht wurde für alle Patentansprüche erstellt.

| Recherchenort | Abschlußdatum der Recherche | Prüfer |
|---|---|---|
| DEN HAAG | 17-08-1983 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82